Europäisches Patentamt

European Patent Office

Office européen des brevets

(11) Veröffentlichungsnummer: **0 470 270 A1**

# EUROPÄISCHE PATENTANMELDUNG

(21) Anmeldenummer: **90114553.2**

(22) Anmeldetag: **30.07.90**

(51) Int. Cl.⁵: **A61B 5/04**, A61B 5/0476

Ein Antrag gemäss Regel 88 EPÜ auf Berichtigung S. 4 Zeile 20; "ausgewählt werden" und Zeitpunkte der räumlichen Quellenaktivität ausgewählt, S. 8 Zeile 6; a,b liegt vor. Über diesen Antrag wird im Laufe des Verfahrens vor der Prüfungsabteilung eine Entscheidung getroffen werden (Richtlinien für die Prüfung im EPA, A-V, 2.2).

(43) Veröffentlichungstag der Anmeldung:
**12.02.92 Patentblatt 92/07**

(84) Benannte Vertragsstaaten:
**AT BE CH DE DK ES FR GB GR IT LI NL SE**

(71) Anmelder: **SIEMENS AKTIENGESELLSCHAFT**
**Wittelsbacherplatz 2**
**W-8000 München 2(DE)**

(72) Erfinder: **Abraham-Fuchs, Klaus,Dipl.-Phys.**
**Graslitzer Strasse 17**
**W-8520 Erlangen(DE)**
Erfinder: **Kohs, Siegfried Kohs, Dipl.Inform.**
**Weintraubengasse 1**
**W-8500 Nürnberg(DE)**
Erfinder: **Prihoda, Heinz, Dipl.-Inform.**
**Lerchenstrasse 12**
**W-8521 Grossenseebach(DE)**
Erfinder: **Uebler, Johann Dipl.-Phys.**
**Ringstrasse 25**
**W-8523 Baiersdorf(DE)**

(54) **Verfahren zur Auswahl, Darstellung und graphischen Umsetzung von räumlich lokalisierbaren biomagnetischen oder bioelektrischen Signalen auf einem Graphikbildschirm.**

(57) Ein Graphikbildschirm ist in mehrere Darstellungsfelder aufgeteilt, von denen ein erstes Feld (2) das betreffende Organ in mehreren orthogonalen Querschnitten (sagittal, axial, coronal) zeigt, denen je ein Organfeld (5, 6, 7) zugeordnet ist, um darin die Wanderungskurve (20) der in den betreffenden Körperorgan wirkenden Quelle aufgrund der von dieser erzeugten und zu bestimmten Meßzeitpunkten gemessenen, elektronisch gespeicherten Aktivitätswerte als Raumkurve darzustellen, und in einem zweiten Feld (3) die biomagnetischen oder bioelektrischen Signale ausgewählter Meßkanäle als Zeit-Amplitudenkurve erscheinen und schließlich in einem dritten Feld (4) der Programmsteuerung dienende, anwählbare alphanumerische Zeichen und/oder Symbole darstellbar sind und zwischen den in den Feldern (2, 3, 4) dargestellten Werten mit Hilfe von Cursoren (14, 15, 16, 17) räumliche, zeitliche und intensitätsbezogene Korrelationen rechnergesteuert darstellbar sind.

EP 0 470 270 A1

FIG 1

Die Erfindung betrifft ein Verfahren zur Auswahl, Darstellung und graphischen Umsetzung von räumlich und zeitlich lokalisierbaren biomagnetischen oder bioelektrischen Signalen auf einem Graphikbildschirm.

Eine Einrichtung und ein Verfahren zur Messung von schwachen orts- und zeitabhängigen Magnetfeldern sind aus der EP-A-0 359 864 bekannt. Dabei geht es darum, biomagnetische oder bioelektrische Meßdaten zur räumlichen Lokalisierung einer physiologischen Aktivität im biologischen Gewebe zu einem definierten Zeitpunkt im Zeitsignal auszuwerten. Dabei erhält man ein Ergebnis in fünf Dimensionen, nämlich die Projektion der Wanderungskurve der Quelle einer Aktivität als dreidimensionale Raumkurve in einer schematischen Darstellung des betreffenden Organs, entlang derer sich die Quelle in der Zeit als vierte Dimension bewegt und als fünfte Dimension die Stärke der Quelle zu einem bestimmten Zeitpunkt in einer geeigneten Meßzahl (z.B. Stromstärke, Volumenstromdichte etc.).

Aus "Med. and Biol. Eng. and Comp." 9/1990 sowie "The Int. Conf. on Biomagnetism", New York, 15-18 Aug. 1989 "Application of a Biomagnetic Multichannal System to the comparativ localization of accessory conduction pathways in patients with WPW Syndrom" ist es bekannt, die Form der Raumkurve als Projektion der Kurve in einer Ebene, jeweils nebeneinander in drei aufeinander orthogonalen Projektionen darzustellen. Diese Projektionen konnten der Anatomie in der Weise zugeordnet werden, daß die entsprechenden orthogonalen Schnittebenen eines anatomischen Abbildungsverfahrens z.B. durch CT oder MR gewonnenen Schnittbildern überlagert wurden. Die zeitliche Information und die Zuordnung zum Zeitsignal wurde durch Beschriftung neben den Punkten auf der räumlichen Kurve erhalten. Dieses Verfahren ist umständlich und zeitaufwendig.

Der Erfindung liegt die Aufgabe zugrunde, eine graphische Darstellung zu finden, die die Informationen in allen fünf Dimensionen für den Arzt auf einfache und übersichtliche Weise auswertbar darzustellen gestattet. Dabei soll der räumliche Verlauf nicht nur abstrakt im Raum, sondern in Relation zu einer räumlichen Abbildung der Anatomie dargestellt werden.

Diese Aufgabe wird durch die im Patentanspruch 1 angegebene Erfindung gelöst. Dadurch ist erreicht, daß auf einem einzigen Bildschirm durch relativ einfache Bedienung mit einer "Maus" eine Vielzahl von an sich abstrakten Signalen zu einer sinnfälligen Information in einer dem Arzt geläufigen anatomischen Darstellung erscheint.

Verbesserungen und weitere Ausbildungen der Erfindung sind in den Unteransprüchen gekennzeichnet.

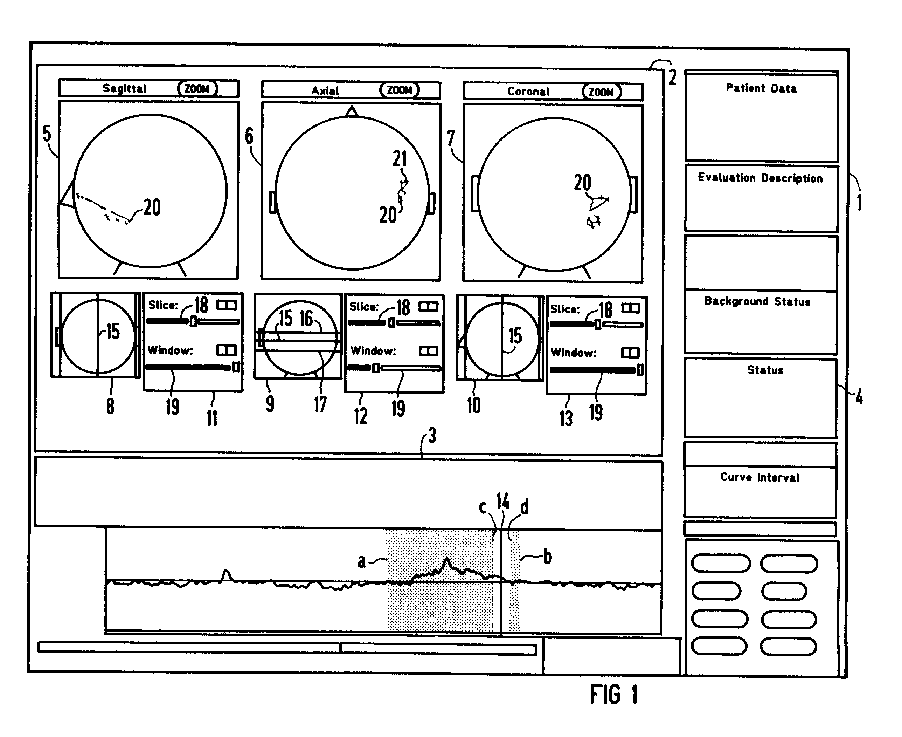

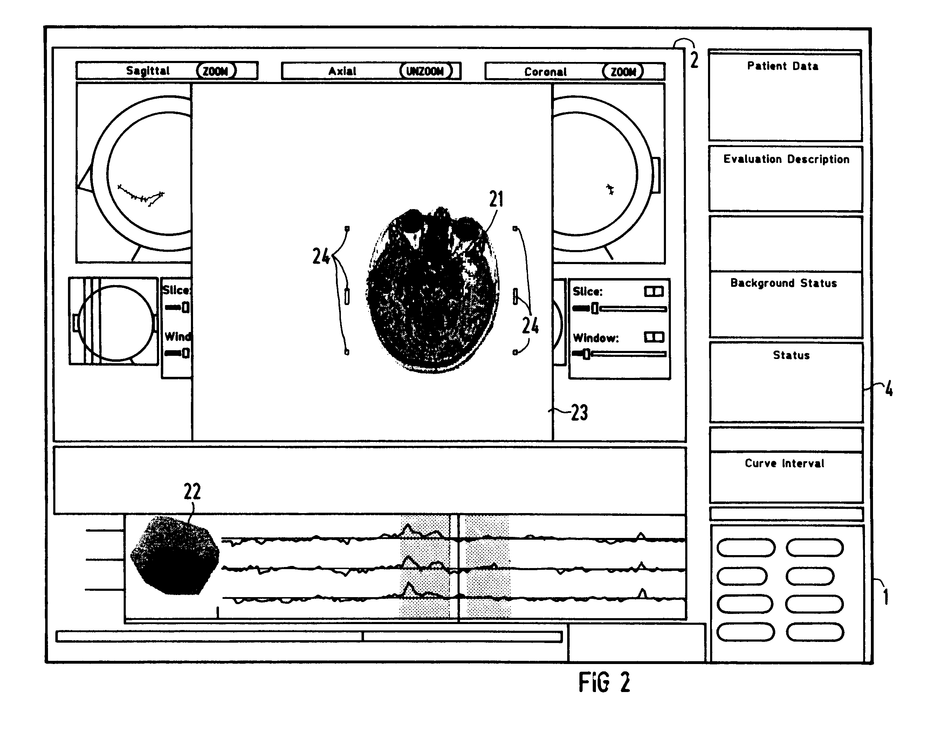

Die Erfindung wird anhand der Fig. 1 und 2 im folgenden auf der Grundlage eines auf einem Bildschirm vereinfacht dargestellten Graphikmusters näher erläutert.

Gemäß Fig. 1 ist der Bildschirm 1 in drei Darstellungsfelder 2, 3, 4 unterteilt. Dabei enthält das Feld 2 drei etwa gleich große, nebeneinander angeordnete Organfelder 5, 6, 7 zur Abbildung von modellhaften oder in schichtbildlicher Darstellung hergestellten Organbildern. Unter diesen sind drei kleinere Symbolfelder 8, 9, 10 angeordnet, die ebenfalls das in den zugeordneten Organfeldern dargestellte Bezugsorgan in verkleinertem Maßstab enthalten, jedoch in einer um jeweils 90° um eine Zentralachse gedrehten Darstellung. Den Symbolfeldern sind Anzeigefelder 11, 12, 13 zugeordnet, die dazu dienen, die in den Symbolfeldern jeweils eingestellten und graphisch dargestellten geometrischen Zustände graphisch oder numerisch anzuzeigen.

Das Feld 3 dient dazu, die von den einzelnen Meßkanälen während der Messung abgegebenen und gespeicherten Signalverläufe über die Zeit darzustellen, wobei eine Anzahl von Kanälen wählbar ist.

Das Bildfeld 4 ist als Bedienfeld für alphanumerische Zeichen vorgesehen, die dazu dienen, die jeweils gewünschten Funktionen und Daten anzusteuern, die auf dem Bildschirm erscheinen, und diese gegebenenfalls zu parametrieren oder zu ändern.

In den Organfeldern 5, 6, 7 ist ein Schädelumriß modellhaft dargestellt, der im Organfeld 5 in sagittaler, im Organfeld 6 in axialer und im Organfeld 7 in coronaler Projektion gezeigt ist. Dieser Schädelumriß ist in den Symbolfeldern 8, 9, 10 gegenüber denjenigen der zugeordneten Organfelder 5, 6, 7 um jeweils 90° um eine senkrechte bzw. waagerechte Zentralachse gedreht, um eine Tiefeninformation zu gewinnen.

Die Auswertung und Darstellung der gespeicherten biomagnetischen oder bioelektrischen Meßdaten kann nun auf folgende Weise geschehen:

Mit Hilfe der "Maus" werden in an sich bekannter Weise über einen im Bildschirm erscheinenden Pfeil die im Bildfeld 4 vorhandenen alphanumerischen Daten angesteuert, die auf dem Bildschirm zur Darstellung kommen sollen. Damit können auch die Parameter bestimmt werden, nach denen die eingehenden Signale ausgewertet und dargestellt werden sollen. Dabei sind sowohl die räumlichen wie die zeitlichen Parameter auswählbar, deren Darstellung gewünscht wird. Die im Bildfeld 4 dargestellten Bezeichnungen sind nur beispielhaft angeführt und können durch andere Zeichen und Symbole ersetzt werden, die beispielsweise sequentiell abrufbar sind.

Die Auswertung der aus den einzelnen Kanälen eingehenden Zeitsignale kann beispielsweise damit beginnen, daß die Kurvenzüge der Signale aller oder einzelner Kanäle anstelle der dargestellten Graphik auf den Bildschirm geholt werden. Diese Kurven können beispielsweise durch Zooming zeitlich gedehnt oder gestaucht werden. Mit Hilfe eines senkrecht über den Bildschirm laufenden Striches, der eine Cursor-Funktion erfüllt, können dann aus einzelnen Kurven bestimmte Zeitpunkte ausgewählt und der Kurvenzug zwischen den Zeitpunkten vergrößert dargestellt werden. Diejenige Kurve oder diejenigen Kurventeile, die sich aufgrund bestimmter Kriterien von einer "Norm" abheben, können dann, nach Umschaltung auf die in Fig. 1 dargestellte Graphik, in das Bildfeld 3 eingeblendet werden. Diejenigen Kurventeile, die als Normabweichung angesehen werden, können dann in dem gestrichelt dargestellten Lokalisierungsbereich zwischen den Kanten a und b eingegrenzt werden. Nun besteht die Möglichkeit, durch die Wahl von elektrischen Parametern, beispielsweise das Signal-Rauschverhältnis oder die Maximalamplitude oder andere Werte aus dem gesamten Lokalisationsbereich "sinnvolle" Bereiche einzugrenzen. Ein solcher Bereich ist der weiße Bereich innerhalb des schraffierten Bereiches mit den Kanten c,d. Innerhalb dieses sinnvollen Bereiches können dann mit Hilfe eines Zeitcursors 14 beliebige Zeitpunkte auszuwählen, die auf noch zu beschreibende Weise in den Organdarstellungen punktuell hervorgehoben darstellbar sind.

Die Erzeugung biomagnetischer Signale dient ja in erster Linie dazu, die Quellen der an unterschiedlichen Raumpunkten gleichzeitig gemessenen magnetischen Feldstärken innerhalb des betreffenden Organs zu lokalisieren und in einer dem Arzt geläufigen Art und Weise räumlich darzustellen. Wichtig dabei sind auch die zeitlichen Änderungen, d.h. die zeitabhängige räumliche Wanderung der Quelle innerhalb des Organs sowie die Stärke der Quelle ebenfalls in Abhängigkeit von Raum und Zeit.

Um nun eine solche Darstellung zu realisieren, muß das Objektbild in mehreren Ebenen erscheinen. Darüber hinaus muß es möglich sein, die räumliche Tiefe des jeweiligen Ereignisses zu lokalisieren. Diese Möglichkeit bieten die verkleinerten Darstellungen der Organbilder in den Symbolfeldern 8, 9, 10. Wie im Symbolfeld 9 gezeigt, werden dazu drei Cursoren 15, 16, 17 als Raumcursoren benötigt, wobei der Cursor 15 die Tiefenebene und die Cursoren 16, 17 die Schichtdicke bestimmen. Diese Raumcursoren sind mit Hilfe der "Maus" in ihrer räumlichen Lage verschiebbar. Die jeweilige räumliche Lage wird mit Hilfe von Symbolen in den Anzeigefeldern 11, 12, 13 dargestellt, und zwar zeigen die Balken 18 die Tiefenlage und die Balken 19 die Schichtgrenzen an. Um diese Werte reproduzierbar zu machen, werden die betreffenden Werte zusätzlich numerisch angezeigt.

Wenn nun die Wahl von Tiefenlage und Schichtdicke getroffen ist, wird die räumliche Bewegung der Quelle, die diese innerhalb der gewählten Schicht im sinnvollen Zeitbereich zwischen den Linien c und d im Lokalisierungsbereich a, b durchläuft, gespeichert und erscheint als Wanderungskurve 20 in den Organfeldern 5, 6, 7.

Nun besteht darüber hinaus die Möglichkeit, die Lage der Quelle auf der Wanderungskurve 20 zu bestimmten Zeitpunkten durch Bewegen des Zeitcursors 14 innerhalb der mit den Raumcursoren 15, 16, 17 gewählten räumlichen Schnitte zu verfolgen. Zu diesem Zweck wird zunächst der betreffende Raum mit Hilfe der Raumcursoren 15, 16, 17 vorgewählt. Sodann wird mit dem Zeitcursor 14 derjenige Zeitpunkt bestimmt, zu dem die räumliche Lage der Quelle gesucht wird. Dieser erscheint dann auf der gespeicherten Wanderungskurve 20 als herausgehobener, vorzugsweise andersfarbiger Punkt 21. In Umkehrung dieser Möglichkeit kann auch der Raumpunkt bestimmt werden, an dem die Quelle zu einem bestimmten Zeitpunkt aktiv war. Dazu wird der Zeitcursor 14 auf einen bestimmten Zeitpunkt eingestellt und die Raumcursoren 15, 16, 17 werden so lange verstellt, bis der herausgehobene Punkt 21 auf der Wanderungskurve 20 erscheint und damit räumlich lokalisiert ist. Durch die Bewegung des Zeitcursors 14 ist also die Möglichkeit eröffnet, die Propagation der Quelle genau zu verfolgen.

Eine für den auswertenden Arzt besonders sinnfällige und exakte Methode der Auswertung ergibt sich daraus, daß die Organfelder 5, 6, 7 statt mit Modellorganen mit echten Schichtbildern belegt werden können, die auf bekannte Weise mit Röntgen- oder NMR-Methoden gewonnen werden. Dies ist in Fig. 2 dargestellt. Die Lokalisierung der einzelnen Schichtbilder zu der biomagnetischen oder bioelektrischen Messung geschieht auf bekannte Weise mit Hilfe einer dem Patienten zugeordneten, mit einer Beißplatte versehenen bekannten Hilfsvorrichtung, die mit sich auf den Röntgenbildern abbildenden Markierungen 24 versehen ist. Die einzelnen Schichten sind numeriert und können mit Hilfe der Anzeigebalken 18 gewählt werden. Auf diese Weise besteht die Möglichkeit, die gemäß Fig. 1 in die Organbilder eingeblendeten Wanderungskurven und -punkte unmittelbar in das betreffende Schichtbild zu übertragen und sichtbar zu machen. Durch die Anwahl der aufeinanderfolgenden Bilder einer Schichtserie wird die notwendige Tiefeninformation erreicht. Der Maßstab der Schichtbilder kann durch Zooming verändert werden. Auf diese Weise ist eine besonders genaue Lokalisation in anatomischer Echtdarstellung möglich.

Gemäß Fig. 2 kann in das Bildfeld 1 neben den Kurvenzügen der einzelnen Meßkanäle auch noch ein Meßbild 22 eingeblendet werden, welches einen Querschnitt der Meßebene darstellt und symbolhaft die zu einem bestimmten Zeitpunkt vorhandene Feldverteilung anzeigt. Dabei kann die Polarität der Feldlinien durch die Farbe und die Feldstärke durch die Farbsättigung dargestellt sein. Im Schwarz-Weiß-Bild sind diese Größen entweder durch Linien gleicher Feldstärke oder durch unterschiedliche Grauwerte mit Anzeige der Polarität Plus, Minus charakterisierbar.

**Patentansprüche**

1. Verfahren zur Auswahl, Darstellung und graphischen Umsetzung von räumlich und zeitlich lokalisierbaren biomagnetischen oder bioelektrischen Signalen auf einem interaktiv bedienbaren Graphikbildschirmen, **dadurch gekennzeichnet,** daß der Bildschirm (1) in mehrere Darstellungsfelder aufgeteilt ist, von denen ein erstes Feld (2) das betreffende Organ in mehreren orthogonalen Querschnitten (sagittal, axial, coronal), denen je ein Organfeld (5, 6, 7) zugeordnet ist, modellhaft oder in schichtbildlicher Echtdarstellung zeigt, um darin die Wanderungskurve (20) der in dem betreffenden Körperorgan wirkenden Quelle aufgrund der von dieser erzeugten und zu bestimmten Meßzeitpunkten gemessenen, elektronisch gespeicherten Aktivitätswerte als Raumkurve darzustellen und in einem zweiten Feld (3) die biomagnetischen oder bioelektrischen Signale ausgewählter Meßkanäle als Zeit-Amplitudenkurve erscheinen und schließlich in einem dritten Feld (4) der Programmsteuerung dienende, anwählbare alphanumerische Zeichen und/oder Symbole darstellbar sind und zwischen den in den Feldern (2, 3, 4) dargestellten Werten mit Hilfe von Cursoren räumliche, zeitliche und intensitätsbezogene Korrelationen rechnergesteuert darstellbar sind.

2. Verfahren nach Anspruch 1, **dadurch gekennzeichnet,** daß die räumliche Lage der Schicht und deren Stärke innerhalb des Organbildes zur Darstellung der dort aktiven Signalquellen mit Hilfe von Raumcursoren (15, 16, 17) wählbar ist.

3. Verfahren nach Anspruch 2, **dadurch gekennzeichnet,** daß den Organfeldern (5, 6, 7) Symbolfelder (8, 9, 10) mit um 90° um die Zentralachse gedrehten und gekippten Hilfsmodelldarstellungen zugeordnet sind, auf denen die gewählte Schichtstärke und deren Mittelage zum Modellorgan erkennbar ist.

4. Verfahren nach einem der vorhergehenden Ansprüche, **dadurch gekennzeichnet,** daß für die im zweiten Feld (3) dargestellten Zeit-Amplitudenkurven Lokalisierungsbereiche (a, b) festlegbar sind und durch Auswahl von Parametern nach unterschiedlichen Kriterien innerhalb der Lokalisierungsbereiche (k, p) sinnvolle Bereiche (c, d) zeitlich eingrenzbar sind, und die diesen Bereichen entsprechenden Quellenbewegungen als Wanderungskurve (20) in den Organbildern erscheint.

5. Verfahren nach Anspruch 4, **dadurch gekennzeichnet,** daß mit Hilfe eines Zeitcursors (14) innerhalb der sinnvollen Bereiche (c, d) Zeitpunkte räumlicher Quellenaktivität auswählbar sind, die als besonders markierte Raumpunkte (21) auf den Wanderungskurven (20) hervorgehoben erscheinen.

6. Verfahren nach einem der vorhergehenden Ansprüche, **dadurch gekennzeichnet,** daß zur Organdarstellung mit Hilfe eines Röntgen- oder NMR-Gerätes erzeugte Schnittbilder des betreffenden Organs benutzt sind, die aufgrund einer dem Patienten zugeordneten Justiervorrichtung mit Justiermarkierungen, die auf dem Röntgen- bzw. NMR-Bild erscheinen, der Quelle der biomagnetischen oder bioelektrischen Signale exakt zuordbar sind.

7. Verfahren nach einem der vorhergehenden Ansprüche, **dadurch gekennzeichnet,** daß parallel zu der jeweils ausgewählten Zeit-Amplitudenkurve eine die Stärke der Quelle im sinnvollen Bereich (c, d) repräsentierende Kurve dargestellt wird.

8. Verfahren nach Anspruch 7, **dadurch gekennzeichnet,** daß parallel zu der jeweils ausgewählten Zeit-Amplitudenkurve eine das Zuverlässigkeitsmaß der Messung im sinnvollen Bereich (c, d) repräsentierende Kurve dargestellt wird.

9. Verfahren nach einem der vorhergehenden Ansprüche, **dadurch gekennzeichnet,** daß in das zweite Feld neben den Zeit-Amplitudenkurven ein Meßbild (22) des Meßquerschnittes eingeblendet ist, welches symbolhaft die zu bestimmten Zeitpunkten in der Meßebene vorhandene Feldverteilung anzeigt, wobei die Polarität der Feldlinien durch die Farbe und die Feldstärke durch die Farbsättigung charakterisiert wird.

FIG 1

FIG 2

EP 0 470 270 A1

**Europäisches Patentamt**

**EUROPÄISCHER RECHERCHENBERICHT**

Nummer der Anmeldung

**EP 90 11 4553**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 355 506 (SIEMENS A.G.)<br>* Seite 3, Zeile 24 - Seite 4, Zeile 41; Figur 1 *<br>– – – | 1,6 | A 61 B 5/04<br>A 61 B 5/0476 |
| A,D | EP-A-0 359 864 (SIEMENS A.G.)<br>* Spalte 9, Zeile 21 - Spalte 10, Zeile 18; Figuren 6,7 *<br>– – – | 1,6 | |
| A | US-A-4 328 491 (M.C. DEMETRESCU et al.)<br>* Spalte 1, Zeile 50 - Spalte 2, Zeile 22; Figur 1 *<br>– – – | 1 | |
| A | ELECTRO MEDICA Band 57, Nr. 1, 1989, Seiten 2-7, Erlangen, DE; F. GUDDEN et al.: "Ein Vielkanalsystem zur Biomagnetischen Diagnostik in Neurologie und Kardiologie: Prinzip, Methode und erste Ergebnisse"<br>* Seite 6, linke Spalte , Zeile 3 - rechte Spalte, Zeile 22; Figuren 1,2,7 *<br>– – – – – | 1 | |
| | | | **RECHERCHIERTE SACHGEBIETE (Int. Cl.5)**<br><br>A 61 B 5/00<br>G 06 F 15/00 |

Der vorliegende Recherchenbericht wurde für alle Patentansprüche erstellt

| Recherchenort | Abschlußdatum der Recherche | Prüfer |
|---|---|---|
| Berlin | 18 März 91 | WEIHS J.A. |